# EUROPEAN PATENT APPLICATION

(11) **EP 4 218 796 A1**
(43) Date of publication of application: **02.08.2023**
(21) Application number: 22154002.4
(22) Date of filing: 28.01.2022
(51) Int. Cl.: A61K 38/48, C12N 9/48, A61P 31/04, A61P 17/00, A61P 17/10

(54) **ANTIBACTERIAL PROPERTIES OF POLYPEPTIDES WITH NLPC/P60 DOMAINS**

(71) Applicant: PHILIPPS-UNIVERSITÄT MARBURG, 35037 Marburg (DE)
(72) Inventor: Graumann, Peter, 35043 Marburg (DE); Fiedler, Svenja, 35043 Marburg (DE)
(74) Representative: Durm Patentanwälte PartG mbB

(57) **Abstract**

The invention relates to a polypeptide (e.g. comprised in a composition or medical device) for use as a medicament, the polypeptide, such as LytF, comprising an NlpC/P60 domain but no CHAP domain of LysK. The composition may further be used as an anti-infectiva such as an antibiotic in particular to treat gram-positive bacterial infections and/or disorder or disorders of the skin (e.g. formulated for epicutaneous administration). The invention further relates to a polynucleotide encoding the polypeptide described herein e.g. comprised in a vector or a host cell such as a bacterial cell. The polypeptide described herein may further be used in a disinfection solution, in packaging material and/or in biotechnological processes.

## Description

The invention relates to a polypeptide (e.g. comprised in a composition or medical device) for use as a medicament, the polypeptide, such as LytF, comprising a NIpC/P60 domain, but no CHAP domain of LysK. The composition may further be used as an anti-infectiva such as an antibiotic in particular to treat gram-positive bacterial infections and/or disorder or disorders of the skin (e.g. formulated for epicutaneous administration). The invention further relates to a polynucleotide encoding the polypeptide described herein e.g. comprised in a vector or a host cell such as a bacterial cell. The polypeptide described herein may further be used in a disinfection solution, in packaging material and/or in biotechnological processes.

Bacterial pathogens represent a significant threat for human health. Although various types of agents having bactericidal or bacteriostatic activity are known in the art (e.g. antibiotics), microbial resistance to these, in particular to antibiotics, is steadily increasing.

Bacteria possess so-called endolysins, which adjust the pore size of the bacterial cell wall to allow length growth of the bacteria by new insertion of cell wall material. The cell wall is under high tension due to the internal pressure of the cell, this is may be regulated by endolysins in the wall to achieve a balance between stability of the wall and its expansion.

There is still a constant need for new antibacterial agents. Preferably, said agents show reduced incidence of resistant strain development while having in parallel a high antibacterial activity, as reflected by a low minimum inhibitory concentration (MIC).

Thus, there is a need for improved antibacterial means and methods.

The above technical problem is solved by the embodiments disclosed herein and as defined in the claims.

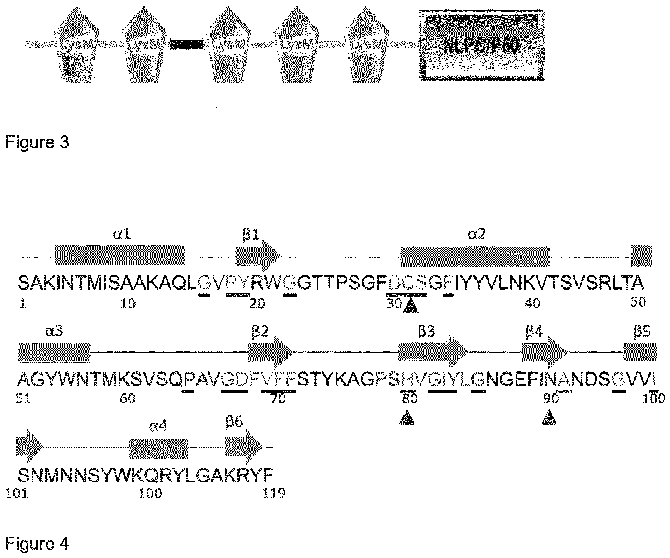

Accordingly, the invention relates to, inter alia, the following embodiments:
1. A composition comprising a polypeptide for use as a medicament, the polypeptide comprising:
   a) an NIpC/P60 domain; and
   b) no CHAP domain of LysK.
2. The composition for use of embodiment 1, wherein the invariant DCS sequence is located at position 30 of the NIpC/P60 domain or an equivalent of position 30 of the NIpC/P60 domain.
3. The composition for use of embodiment 1 or 2, wherein the NIpC/P60 domain described herein comprises the DCS sequence embedded in a sequence part
   a) selected from the group consisting of
      GFDCSGF (SEQ ID NO: 15), GIDCSGF (SEQ ID NO: 16), GFDCSAF (SEQ ID NO: 17), GIDCSAF (SEQ ID NO: 18), AFDCSGF (SEQ ID NO: 19), AIDCSGF (SEQ ID NO: 20), AFDCSAF (SEQ ID NO: 21), AIDCSAF (SEQ ID NO: 22), GLDCSGF (SEQ ID NO: 39), GLDCSAF (SEQ ID NO: 40), ALDCSGF (SEQ ID NO: 41) and ALDCSAF (SEQ ID NO: 42); or
   b) a sequence part according (a) wherein 1, 2 or 3 amino acid(s) is/are inserted, deleted or substituted.
4. The composition for use of any one of embodiments 1 to 3, wherein the NIpC/P60 domain described herein comprises adjacent to on beta 2:
   a) a sequence as defined by VGDFVFF (SEQ ID NO: 24) or sequence as defined by VGDFVFF (SEQ ID NO: 24), wherein 1, 2 or 3 amino acid(s) is/are inserted, deleted or substituted; or
   b) a sequence as defined by VGDLVFF (SEQ ID NO: 24) or a sequence as defined by VGDLVFF (SEQ ID NO: 25), wherein 1, 2 or 3 amino acid(s) is/are inserted, deleted or substituted.
5. The composition for use of any one of embodiments 1 to 4, wherein the NIpC/P60 domain described herein comprises at least partially on beta strand 3:
   a) a sequence selected from the group consisting of:
      HVGIYLG (SEQ ID NO: 26), HMGIYLG (SEQ ID NO: 27), HVGIYIG (SEQ ID NO: 28), HVGIYLN (SEQ ID NO: 29), HMGIYIG (SEQ ID NO: 30), HMGIYLN (SEQ ID NO: 31) and HVGIYIN (SEQ ID NO: 32); or
   b) a sequence part according (a) wherein 1, 2, 3 or 4 amino acid(s) is/are inserted, deleted or substituted.
6. The composition for use of any one of embodiments 1 to 5, wherein the NlpC/P60 domain described herein comprises at least partially on beta strand 4:
   a) a sequence selected from the group consisting of:
      FINA (SEQ ID NO: 33), FVNA (SEQ ID NO: 34), FANA (SEQ ID NO: 35), FIHA (SEQ ID NO: 36), FVHA (SEQ ID NO: 37) and FAHA (SEQ ID NO: 38); or
   b) a sequence part according (a) wherein 1 or 2 amino acid(s) is/are inserted, deleted or substituted.
7. The composition for use of any one of embodiments 1 to 6, wherein the NlpC/P60 domain described herein comprises or consists of a sequence as defined by SEQ ID NO: 1 or a sequence having at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 99% sequence identity to SEQ ID: 1.
8. The composition for use of embodiment 7, wherein the amino acids underlined in Figure 4 are conserved.
9. The composition of any one of embodiments 1 to 8 for use as an anti-infective medicament.
10. The composition for use of embodiment 9, wherein the anti-infective medicament is an antibiotic medicament.
11. The composition for use of embodiment 10, wherein the antibiotic medicament is used in the treatment and/or prevention of a gram-positive bacterial infection.
12. The composition of any one of embodiments 1 to 8 for use in treatment of a disease or disorder of the skin.
13. The composition for use of embodiment 12, wherein the disease or disorder of the skin is caused by at least one cause or disease selected from the group consisting of: acneiform eruptions, autoinflammatory syndromes, chronic blistering, conditions of the mucous membranes, conditions of the skin appendages, conditions of the subcutaneous fat, congenital anomalies, connective tissue diseases, abnormalities of dermal fibrous, abnormalities of elastic tissue, dermal and subcutaneous growths, dermatitis, drug eruptions, endocrine-related disease, epidermal nevi, epidermal neoplasms, epidermal cysts, erythemas, genodermatoses, lichenoid eruptions, lymphoid-related disease, melanocytic nevi, neoplasms, monocyte- and macrophage-related disease, mucinoses, neurocutaneous, noninfectious immunodeficiency-related disease, papulosquamous hyperkeratotic, pregnancy, pruritic, psoriasis, reactive neutrophilic disease, recalcitrant palmoplantar eruptions, metabolic disease, physical factors, urticaria, angioedema and vascular-related diseases.
14. The composition for use of embodiment 1 to 13, wherein the polypeptide is LytF or a derivative, variant or analogue thereof.
15. The composition for use of embodiments 1 to 14, wherein the composition is formulated for epicutaneous administration.
16. The composition for use of embodiment 15, wherein the composition is formulated for a galenic form selected from the group consisting of: plaster, compress, topical solution, topical emulsion, topical suspension, cream, ointment, paste and gel.
17. A medical device comprising a polypeptide comprising:
   a) an NIpC/P60 domain; and
   b) no CHAP domain of LysK.
18. The medical device of embodiment 17, wherein the medical device is a device selected from the group consisting of: implant, stent, catheter, intrauterine device, plaster, compress and dressing.
19. A polynucleotide comprising or consisting of a sequence as defined by the SEQ ID NO: 3 or a sequence having 25% sequence identity thereof, encoding at least one polypeptide comprising:
   a) an NIpC/P60 domain; and
   b) no CHAP domain of LysK,
   preferably LytF.
20. A vector comprising the polynucleotide of embodiment 19.
21. A host cell comprising the polynucleotide of embodiment 19.
22. The host cell of embodiment 21, wherein the host cell is a bacterial cell.
23. A disinfection solution comprising a polypeptide, the polypeptide comprising:
   a) an NlpC/P60 domain; and
   b) no CHAP domain of LysK.
24. A packaging material comprising a polypeptide, the polypeptide comprising:
   a) an NIpC/P60 domain; and
   b) no CHAP domain of LysK,
   preferably LytF.
25. A method for the production of a polypeptide, the method comprising the steps of:
   i) culturing the host cell of embodiment 21 or 22; and
   ii) isolating the polypeptide comprising:
      a) an NIpC/P60 domain; and
      b) no CHAP domain of LysK.
26. A method for conservation of a product, the method comprising adding at least one polypeptide to a product, the polypeptide comprising:
   a) an NIpC/P60 domain; and
   b) no CHAP domain of LysK
27. A method for prevention and/or containment of a contamination of a biotechnological process, the method comprising the steps of:
   i) combining with a nutrient a polypeptide, the polypeptide comprising:
      a) an NlpC/P60 domain; and
      b) no CHAP domain of LysK
      for use of preventing and/or containing a contamination; and ii) adding at least one cell to the nutrient medium to initiate and/or proceed the biotechnological process, wherein the cell is at least partially resistant to the polypeptide.
28. The method of embodiment 27, wherein the biotechnological process is the biotechnological production of at least one small molecule, nucleotide, or polypeptide.

Accordingly, in one embodiment, the invention relates to a composition comprising a polypeptide for use as a medicament, the polypeptide comprising: a) an NIpC/P60 domain; and b) no CHAP domain of LysK.

The term "polypeptide", as used herein, refers to a polymer of amino acids linked by peptide bonds in a specific sequence. The amino acid residues of a polypeptide may be modified by e.g. covalent attachments of various groups such as carbohydrates and phosphate. Other substances may be more loosely associated with the polypeptide, such as heme or lipid, giving rise to conjugated polypeptides which are also comprised by the term "polypeptide" as used herein. The term as used herein is intended to encompass also proteins. Thus, the term "polypeptide" also encompasses for example complexes of two or more amino acid polymer chains. The term "polypeptide" does encompass embodiments of polypeptides which exhibit optionally modifications typically used in the art, e.g. biotinylation, acetylation, pegylation, chemical changes of the amino-, SH- or carboxyl-groups (e.g. protecting groups) etc. As will become apparent from the description below, the polypeptide according to the present invention is a non-naturally occurring polypeptide. The term "polypeptide", as used herein, is not limited to a specific length of the amino acid polymer chain, but typically the polypeptide will exhibit a length of more than 250 amino acids. Usually, but not necessarily, a typical polypeptide of the present invention will not exceed about 750 amino acids in length, preferably not exceed about 500 amino acids in length.

The term "variant", as used herein, refers to an agent having an amino acid sequence which exhibits, in comparison to the respective reference sequence, one or more additions, deletions, insertions, substitutions and/or combinations thereof. This includes for example combinations of deletions/insertions, insertions/deletions, deletions/additions, additions/deletions, insertion/ additions, additions/insertions etc. A person skilled in the art will however understand that the presence of an amino acid residue at a certain position of the variant sequence which is different from the one that is present at the respective same position in the reference sequence is not a combination of, for example, a deletion and a subsequent insertion at the same position but is a substitution as defined herein. Rather, if reference is made herein to combinations of one or more of additions, deletions, insertions, and substitutions, then combination of changes at distinct positions in the sequence are intended, e.g. an addition at the N-terminus and an intrasequential deletion. Such derived sequence will exhibit a certain level of sequence identity with the respective reference sequence, for example a given SEQ ID NO: 6, which is preferably at least 80%, at least 85%, at least 90%, at least 95%, at least 96%, at least 97%, at least 98%, at least 99% or at least 99.5%. Preferred variant sequences are fragments of the parent molecule, for example a given variant (e.g. having a sequence defined by SEQ ID NO: 7), retaining the activity of the parent molecule (e.g. having a sequence defined by SEQ ID NO: 6), i.e. exhibiting on a general level same activity as the respective parent molecule. However, said activity can be the same, higher or lower as the respective parent molecule. Also preferred variant sequences are those resulting from conservative amino acid substitutions within the parent sequence, for example a given SEQ ID NO: 6, again retaining the activity of the parent molecule on a general level.

"Percent (%) sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared.

The term "NlpC/P60 domain", as used herein, refers to a domain comprising or consisting of
a) a folding pattern alpha helix 1/beta sheet 1/alpha helix 2/alpha helix 3/beta sheet 2/beta sheet 3/beta sheet 4/beta sheet 5/alpha helix 4/beta sheet 6, in which beta sheet 2/3/4 and 5 form a central beta-sheet structure backed by alpha helix 1, 2 and 3 on one side and alpha helix 4 on the other side, wherein beta sheet 1 is between alpha helix 1 and 2, or between alpha helix 2 and 3 (see folding pattern schematically presented in in Figure 4); and
b) an invariant DCS sequence adjoining the alpha helix 2.

The folding patterns described herein are preferably determined under physiological conditions (pH 7).

The NIpC/P60 domain described herein often comprises a GVPYRWGG (SEQ ID NO: 11) sequence part or a sequence part with high similarity (G - >90% V -> 90% P - Y->90% R or K -> 90%W -> 90%G->90% G or A) following the alpha helix 1. In some embodiments, the NlpC/P60 domain described herein further comprises between alpha helix 1 and alpha helix 2 a sequence part:
a) selected from the group consisting of GVPYRWGG (SEQ ID NO: 11), GVPYRWGA (SEQ ID NO: 12), GVPYKWGG (SEQ ID NO: 13) and GVPYKWGA (SEQ ID NO: 14); or
b) a sequence part according (a) wherein 1, 2, 3 or 4 amino acid(s) is/are inserted, deleted or substituted.

The sequence part between alpha helix 1 and alpha helix 2 described herein preferably is at least partially on the beta sheet 1 structure.

In some embodiments, the invariant DCS sequence is located at position 30 of the NIpC/P60 domain or an equivalent of position 30 of the NIpC/P60 domain. The skilled person is aware how to determine the position 30 of the NlpC/P60 domain, which is located about at the beginning of the alpha helix 2. Determining a position can be achieved in various ways that are within the skill in the art, for instance, using publicly available computer software such as BLAST, BLAST-2, ALIGN or Megalign (DNASTAR) software.

Typically, the DCS sequence is embedded in the sequence GFDCSGF or a sequence with high similarity (e.g. G or rarely A - L, F or I - D-C-S-G or rarely A-F). In some embodiments the NIpC/P60 domain described herein comprises the DCS sequence embedded in a sequence part
a) selected from the group consisting of
   GFDCSGF (SEQ ID NO: 15), GIDCSGF (SEQ ID NO: 16), GFDCSAF (SEQ ID NO: 17), GIDCSAF (SEQ ID NO: 18), AFDCSGF (SEQ ID NO: 19), AIDCSGF (SEQ ID NO: 20), AFDCSAF (SEQ ID NO: 21), AIDCSAF (SEQ ID NO: 22), GLDCSGF (SEQ ID NO: 39), GLDCSAF (SEQ ID NO: 40), ALDCSGF (SEQ ID NO: 41) and ALDCSAF (SEQ ID NO: 42); or
b) a sequence part according (a) wherein 1, 2 or 3 amino acid(s) is/are inserted, deleted or substituted.

Typically, the NIpC/P60 domain described herein comprises adjacent to and on beta sheet 2 comprises the sequence PAVGDFVFF(SEQ ID NO: 23) or a sequence with high similarity to PAVGDFVFF (SEQ ID NO: 23) (>90% P - variable - >90% V - G - D - > 80% F or L - V - > 90% F - F). In some embodiments, the NlpC/P60 domain described herein comprises adjacent to on beta sheet 2:
a) a sequence as defined by VGDFVFF (SEQ ID NO: 24) or sequence as defined by VGDFVFF (SEQ ID NO: 24), wherein 1, 2 or 3 amino acid(s) is/are inserted, deleted or substituted; or
b) a sequence as defined by VGDLVFF (SEQ ID NO: 24) or a sequence as defined by VGDLVFF (SEQ ID NO: 25), wherein 1, 2 or 3 amino acid(s) is/are inserted, deleted or substituted.

Typically, the NIpC/P60 domain described herein comprises an invariant H at position 80 within a sequence HVGIYLG (SEQ ID NO: 26) on beta sheet 3 (H - >90% V or M - G - I - Y - >90% L or I - G - >90% N or G). In some embodiments, the NlpC/P60 domain described herein comprises at least partially on beta sheet 3:
a) a sequence selected from the group consisting of:
   HVGIYLG (SEQ ID NO: 26), HMGIYLG (SEQ ID NO: 27), HVGIYIG (SEQ ID NO: 28), HVGIYLN (SEQ ID NO: 29), HMGIYIG (SEQ ID NO: 30), HMGIYLN (SEQ ID NO: 31) and HVGIYIN (SEQ ID NO: 32); or
b) a sequence part according (a) wherein 1, 2, 3 or 4 amino acid(s) is/are inserted, deleted or substituted.

Typically, the NlpC/P60 domain described herein comprises a sequence FINA (SEQ ID NO: 33) or a sequence highly similar to FINA (SEQ ID NO: 33) (>90% F - > 90% I or V or A - H or N - >90% A) on beta sheet 4. In some embodiments, the NIpC/P60 domain described herein comprises at least partially on beta sheet 4:
a) a sequence selected from the group consisting of:
   FINA (SEQ ID NO: 33), FVNA (SEQ ID NO: 34), FANA (SEQ ID NO: 35), FIHA (SEQ ID NO: 36), FVHA (SEQ ID NO: 37) and FAHA (SEQ ID NO: 38); or
b) a sequence part according (a) wherein 1 or 2 amino acid(s) is/are inserted, deleted or substituted.

In some embodiments, the NIpC/P60 domain described herein comprises or consists of a sequence as defined by SEQ ID NO: 1 or a sequence having at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 99% sequence identity to SEQ ID: 1. In some embodiments, the NIpC/P60 domain described herein comprises or consists of a sequence having at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 99% sequence identity to SEQ ID: 1, wherein the amino acids underlined in Figure 4 are conserved.

NlpC/P60 domains are typically found in cell-wall peptidases, particularly in cell-wall peptidases of bacterial lineages. NlpC/P60 domains and variations therein are described for example in Anantharaman, V., & Aravind, L., 2003, Genome biology, 4(2), R11. Typically, the NIpC/P60 domain enables the peptidase activity.

The term "CHAP domain of LysK", as used herein, refers to cysteine-histidine dependent amido-hydrolase/peptidase domain of LysK according to SEQ ID NO: 2.

The inventors found that the enzymatic activity of the NIpC/P60 domain in absence of the CHAP domain of LysK is effective in treatment without having an intolerable side effect profile. The enzymatic activity of NIpC/P60 domain destabilizes the cell walls in bacteria an may therefore be useful for example as an active ingredient for treatment, for prevention of secondary infections, as an adjuvant therapeutic and/or as a preservative of other active ingredients.

Accordingly, the invention is at least in part based on the surprising enzymatic activity of the NIpC/P60 domain, as described herein.

In some embodiments, the invention relates to the composition of the invention for use according to the invention, wherein the composition comprises at least one further therapeutic agent.

The term "therapeutic agent", as used herein, refers a compound or a composition of matter that upon administration to a subject in a therapeutically effective amount, provides a therapeutic benefit to the subject. A therapeutic agent may be any type of drug, medicine, pharmaceutical, hormone, antibiotic, protein, gene, growth factor, bioactive material, used for treating, controlling, or preventing diseases or medical conditions. Those skilled in the art will appreciate that the term "therapeutic agent" is not limited to drugs that have received regulatory approval. In some embodiments, one or more therapeutic agents is selected from the group consisting of anti-inflammatory agent, immunomodulator, antiviral agent, antifungal agent, antibiotic agent, hydration agent, wound healing promoter and a combination of these agents.

In certain embodiments, the invention relates to the composition of the invention for use as an anti-infective medicament.

The term "anti-infective medicament", as used herein, refers to a medicament that is effective in treating an infective pathogen. The anti-infective medicament may have, inter alia, properties of preventing, inhibiting, suppressing, reducing, adversely impacting, and/or interfering with the growth, survival, replication, function, and/or dissemination of at least one pathogen.

The inventors found that the polypeptide comprised in the composition of the invention can interfere with the structural elements and reproduction of pathogens.

Accordingly, the invention is at least in part based on the anti-infective activity of the NIpC/P60 domain.

In certain embodiments, the invention relates to the composition for use of the invention, wherein the anti-infective medicament is an antibiotic medicament.

The term "antibiotic medicament", as used herein, refers to a medicament that is effective in treating a bacterial infection. The antibiotic medicament may have, inter alia, properties of preventing, inhibiting, suppressing, reducing, adversely impacting, and/or interfering with the growth, survival, replication, function, and/or dissemination of at least one bacterium.

The polypeptide comprised in the composition of the invention was known as an element of the cell wall of bacteria. The inventors found that the polypeptide can interfere with the structural elements and reproduction of bacteria.

Accordingly, the invention is at least in part based on the antibacterial activity of the NIpC/P60 domain.

In some embodiments, the bacterium described herein is a bacterium, wherein the cell wall formation and/or cell wall growth comprises cross-linking by hydrolysis between D-glutamate and diaminopimelic acid.

In certain embodiments, the invention relates to the composition for use of the invention, wherein the antibiotic medicament is used in the treatment and/or prevention of a gram-positive bacterial infection.

The term "treatment" (and grammatical variations thereof such as "treat" or "treating"), as used herein, refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, decreasing the rate of disease progression, amelioration or palliation of the disease state, and remission or improved prognosis.

The term "prevention", as used herein, relates to the capacity to prevent, minimize or hinder the onset or development of a disorder, disease or condition before its onset.

The term "gram-positive bacterial infection", as used herein, refers to a bacterial infection, wherein the bacteria give a positive result in a Gram stain test. Methods to distinguish gram-positive bacteria from other bacteria are known to the person skilled in the art (see e.g. Gregersen, T., 1978, European journal of applied microbiology and biotechnology, 5(2), 123-127).

In some embodiments, the gram-positive bacterial infection described herein is a cocci infection or bacillus infection. In some embodiments, the gram-positive bacterial infection described herein a bacterial infection selected from the group of Bacillus subtilis, Bacillus licheniformis and Bacillus pumilus.

Without being bound by theory, the peptide of the composition of the invention inhibits cross-linking by hydrolysis between D-glutamate and diaminopimelic acid. This cross-linking inhibition results in growth inhibition at least in certain bacteria.

Accordingly, the invention is at least in part based on the antibacterial activity of the NIpC/P60 domain against gram-positive bacteria.

In certain embodiments, the invention relates to the composition of the invention for use in treatment of a disease or disorder of the skin.

The term "disease or disorder of the skin", as used herein, refers to any disease or disorder that affects the integumentary system, preferably disease or disorder that affects the skin itself, more preferably a disease or disorder that affects primarily the skin itself. In some embodiments, the disease or disorder of the skin described herein is a disease or disorder that affects the epidermis. In some embodiments, the disease or disorder of the skin described herein is at least one disease or disorder selected from the group of gene-related disease or disorder, metabolism-related disease or disorder, physical-factor-related disease or disorder, inflammation-related disease or disorder and autoimmune-related disease or disorder.

In certain embodiments, the invention relates to the composition for use of the invention, wherein the disease or disorder of the skin is caused by at least one cause or disease selected from the group consisting of: acneiform eruptions, autoinflammatory syndromes, chronic blistering, conditions of the mucous membranes, conditions of the skin appendages, conditions of the subcutaneous fat, congenital anomalies, connective tissue diseases, abnormalities of dermal fibrous, abnormalities of elastic tissue, dermal and subcutaneous growths, dermatitis, drug eruptions, endocrine-related disease, epidermal nevi, epidermal neoplasms, epidermal cysts, erythemas, genodermatoses, lichenoid eruptions, lymphoid-related disease, melanocytic nevi, neoplasms, monocyte- and macrophage-related disease, mucinoses, neurocutaneous, noninfectious immunodeficiency-related disease, papulosquamous hyperkeratotic, pregnancy, pruritic, psoriasis, reactive neutrophilic disease, recalcitrant palmoplantar eruptions, metabolic disease, physical factors, urticaria, angioedema and vascular-related diseases.

Diseases or disorders of the skin are particularly prone to secondary infections. Furthermore, diseases or disorders of the skin can be treated locally with compounds at doses that would emit an antigenic effect when administered systemically.

Accordingly, the invention is at least in part based on the local antibacterial activity of the NlpC/P60 domain.

In certain embodiments, the invention relates to the composition for use of the invention, wherein the polypeptide comprises at least one, at least two, at least three, at least four or at least five LysM domain(s).

In certain embodiments, the invention relates to the composition for use of the invention, wherein the polypeptide is LytF or a derivative, variant or analogue thereof.

The term "LytF", as used herein, refers to a polypeptide comprising an amino acid sequence as defined by SEQ ID NO: 6. In some embodiments, LytF is a polypeptide comprising an amino acid sequence as defined by SEQ ID NO: 7. In some embodiments, LytF is a polypeptide comprising an amino acid sequence as defined by SEQ ID NO: 5.

The term "derivative", as used herein, refers to a peptide having chemical substitution (e.g., alphamethylation, alpha-hydroxylation), deletion (e.g., deamination), or modification (e.g., N-methylation) of some groups of amino acid residues in the amino acid sequence of the polypeptide described herein. Preferably, the derivative described herein retains the gamma-D-glutamate-meso-diaminopimelate muropeptidase activity of LytF.

The term "analogue", as used herein, refers to a molecule that is not identical, but has analogous functional and/or structural features. For example, a polypeptide analog retains the biological activity of a corresponding naturally-occurring polypeptide, while having certain biochemical modifications that enhance the analog's function relative to a naturally occurring polypeptide. Such biochemical modifications could increase the analog's protease resistance, membrane permeability, or half-life, without altering, for example, gamma-D-glutamate-meso-diaminopimelate muropeptidase activity.

Without being bound by theory, the endolysin LytF is a gamma-D-glutamate-meso-diaminopimelate muropeptidase that destroys cross-linking by hydrolysis between D-glutamate and diaminopimelic acid. At least in bacteria with this type of cross-linking, LytF acts as a bacteriocide.

Accordingly, the invention is at least in part based on the surprising effect of LytF than can be used for therapy.

In certain embodiments, the invention relates to the composition for use of the invention, wherein the composition is formulated for epicutaneous administration.

Methods for the formulation for epicutaneous administration are known to the person skilled in the art (see e.g., Tomoko Maeda-Chubachi, Elizabeth Kernodle Hussey, Sylvia Furst, 2020, Dermatological Drug Development, Cambridge Scholars Publishing, 2020).

In some embodiments, the polypeptide described herein is comprised in the composition in a concentration of at least 0.005 µmol/g, at least 0.01 µmol/g, at least 0.05 µmol/g, at least 0.1 µmol/g, at least 0.5 µmol/g, at least 0.6 µmol/g, at least 0.7 µmol/g, at least 0.8 µmol/g, at least 0.9 µmol/g, at least 1 µmol/g, at least 2 µmol/g, at least 3 µmol/g, at least 4 µmol/g, at least 5 µmol/g, at least 6 µmol/g, at least 7 µmol/g, at least 8 µmol/g, at least 9 µmol/g, at least 10 µmol/g, at least 11 µmol/g, at least 12 µmol/g, at least 13 µmol/g, at least 14 µmol/g, at least 15 µmol/g, at least 20 µmol/g, at least 25 µmol/g or at least 30 µmol/g.

In certain embodiments, the invention relates to the composition for use of the invention, wherein the composition is formulated for a galenic form selected from the group consisting of: plaster, compress, topical solution, topical emulsion, topical suspension, cream, ointment, paste and gel.

The inventors found that the polypeptide described herein is particularly stable and retains its activity. This stability offers prolonged activity and in particular in epicutaneous administration formulation.

Accordingly, the invention is at least in part based on the topical antipathogen activity and stability of the NlpC/P60 domain.

In certain embodiments, the invention relates to a medical device comprising a polypeptide comprising: a) an NIpC/P60 domain; and b) no CHAP domain of LysK.

The inventors found that the polypeptide described herein can be incorporated in medical devices to avoid bacterial growth on the device and/or to induce a local anti-infective effect.

In some embodiments, the invention relates to the medical device of the invention, wherein the medical device is a device for use in a moist environment.

The term "moist environment", as used herein refers to an environment characterized by the presence of liquid, such as water. In some embodiments, the moist environment described herein is an environment with at least 1%, at least 2%, at least 3%, at least 4%, at least 5%, at least 6%, at least 7%, at least 8%, at least 9%, at least 10%, at least 20%, at least 30%, at least 40%, at least 50%, at least 60%, at least 70%, at least 80% water. The liquid in the moist environment described herein can be hindered from evaporation e.g. by a mechanical or physiological cover, a lipid layer or an emulsion. In some embodiments, the moist environment described herein is a moist wound healing environment. In some embodiments, the moist environment is a wet environment. In some embodiments, the liquid in the moist environment described herein is a body fluid. In some embodiments the wet environment described herein is at least one selected from the group consisting of subcutaneous environment, muscular environment, vaginal environment, urethral environment, uterine environment, rectal environment, gastrointestinal environment, peritoneal environment, oral environment, nasal environment, vascular environment, bone environment, spinal environment, cardiac environment, kidney environment, liver environment and intravitreal environment.

The inventors found, that in the presence of a substantial amount liquid, the polypeptide described herein is particularly active.

In certain embodiments, the invention relates to the medical device of the invention, wherein the medical device is a device selected from the group consisting of: implant, stent, catheter, intrauterine device, plaster, compress and dressing.

The inventors found that in certain medical devices, wherein stability is particularly crucial for efficiency, the polypeptide described herein emits a particularly positive effect, amongst others due to the low reduction of the activity of the polypeptide over time. As such the medical devices described herein are useful in the treatment of acute and/or chronic conditions and diseases.

Accordingly, the invention is at least in part based on the topical antipathogen activity and stability of the NIpC/P60 domain.

In some embodiments, the polypeptide described herein is comprised in the medical device or in the composition in a dose to achieve a maximal concentration at the location of action of at least 0.1 µM, at least 0.2 µM, at least 0.3 µM, at least 0.4 µM, at least 0.5 µM, at least 0.6 µM, at least 0.7 µM, at least 0.8 µM, at least 0.9 µM, at least 1 µM, at least 2 µM, at least 3 µM, at least 4 µM, at least 5 µM, at least 6 µM, at least 7 µM, at least 8 µM, at least 9 µM, at least 10 µM, at least 11 µM, at least 12 µM, at least 13 µM, at least 14 µM or at least 15 µM.

In certain embodiments, the invention relates to a polynucleotide comprising or consisting of a sequence as defined by the SEQ ID NO: 3 or a sequence having at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 97%, or at least 99% sequence identity thereof, encoding at least one polypeptide comprising: a) an NIpC/P60 domain; and b) no CHAP domain of LysK.

In certain embodiments, the invention relates to a polynucleotide comprising or consisting of: i) a sequence as defined by the SEQ ID NO: 3 or a sequence having at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 97%, or at least 99% sequence identity thereof and ii) a His tag (e.g. SEQ ID NO: 8), wherein the polynucleotide encodes at least one polypeptide comprising: a) an NIpC/P60 domain; and b) no CHAP domain of LysK.

In certain embodiments, the invention relates to a polynucleotide comprising or consisting of a sequence as defined by the SEQ ID NO: 9 or a sequence having at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 97%, or at least 99% sequence identity thereof, encoding at least one polypeptide comprising: a) an NIpC/P60 domain; and b) no CHAP domain of LysK.

In certain embodiments, the invention relates to a polynucleotide comprising or consisting of a sequence as defined by the SEQ ID NO: 3 or a sequence having at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 97%, or at least 99% sequence identity thereof, encoding LytF or a variant, analogue or derivative thereof.

In certain embodiments, the invention relates to a polynucleotide comprising or consisting of: i) a sequence as defined by the SEQ ID NO: 3 or a sequence having at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 97%, or at least 99% sequence identity thereof and ii) a His tag (e.g. SEQ ID NO: 8), wherein the polynucleotide encodes LytF or a variant, analogue or derivative thereof.

In certain embodiments, the invention relates to a polynucleotide comprising or consisting of a sequence as defined by the SEQ ID NO: 9 or a sequence having at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 97%, or at least 99% sequence identity thereof, encoding LytF or a variant, analogue or derivative thereof.

The term "polynucleotide", as used herein, refers to any kind of deoxyribonucleotide (e.g. DNA, cDNA) or ribonucleotide (e.g. RNA, mRNA) polymer or a combination of deoxyribonucleotide and ribonucleotide (e.g. DNA/RNA) polymer, in linear or circular conformation, and in either single - or double - stranded form. These terms are not to be construed as limiting with respect to the length of a polymer and can encompass known analogues of natural nucleotides, as well as nucleotides that are modified in the base, sugar and/or phosphate moieties (e.g. phosphorothioate backbones). In general, an analogue of a particular nucleotide has the same base-pairing specificity; i.e., an analogue of A will base-pair with T.

"Percent (%) sequence identity" with respect to a reference polynucleotide sequence is defined as the percentage of nucleotides in a candidate sequence that are identical with the nucleotide in the reference polypeptide sequence, after aligning the sequences and introducing gaps, if necessary, to achieve the maximum percent sequence identity, and not considering any conservative substitutions as part of the sequence identity. Alignment for purposes of determining percent polynucleotide sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared.

In certain embodiments, the invention relates to a vector comprising the polynucleotide of the invention.

The term "vector", as used herein, refers to vehicle for a nucleic acid (DNA or RNA) molecule, which contains one or more heterologous nucleic acid sequence(s) of the invention and, preferably, is designed for transfer between different host cells. The vector described herein may be any vector that is effective to incorporate and express one or more polynucleotide described herein, in a cell, preferably under the regulation of a promoter. A cloning or expression vector may comprise additional elements, for example, regulatory and/or post-transcriptional regulatory elements in addition to a promoter. In some embodiments, the vector described herein is a vector for stable transfection. In some embodiments, the vector described herein is a vector for transient transfection.

In some embodiments, the vector described herein is used in gene therapy. In some embodiments, the vector described herein is used for the production of the polypeptide described herein.

In some embodiments, the vector described herein is a plasmid vector such as a pET24d+ vector.

In certain embodiments, the invention relates to a host cell comprising the polynucleotide of the invention.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell but may contain mutations. Mutant progeny that have the same function or biological activity as screened or selected for in the originally transformed cell are included herein.

In certain embodiments the host cell is directly or indirectly used in therapy (e.g., cell therapy).

In certain embodiments a method for cell therapy comprises the steps of (i) obtaining a cell from a subject; (ii) transform the cell using a tool (e.g. a vector) comprising the polynucleotide described herein and/or transform the cell to produce the antibody of the invention; and (iii) administering the transformed cell to a subject.

In certain embodiments, the host cell is a stem cell. In other embodiments, the host cell is a differentiated cell.

In some embodiments, the polynucleotide described herein is transfected to the host cell with the support of a transfection enhancer, e.g., a transfection enhancer selected from the group of oligonucleotides, lipoplexes, polymersomes, polyplexes, dendrimers, inorganic nanoparticles and cell-penetrating peptides.

In a certain embodiment the invention relates to a method for producing a polypeptide comprising culturing the host cell of the invention, wherein the host cell comprises the polynucleotide of the invention. In a particular embodiment, the method of producing a polypeptide comprises culturing the host cell of the invention under conditions suitable to allow efficient production of the polypeptide described herein.

In certain embodiments, the invention relates to the host cell of the invention, wherein the host cell is a bacterial cell.

The inventors found that the polypeptide described herein can efficiently be produced in certain bacterial host cells, despite the antibacterial properties of the polypeptide.

The polypeptide described herein can be designed such that it does not or not substantially reaches the cell wall of the producing cell. This can be achieved for example by an omitted, removed or dysfunctional signal peptide.

In some embodiments, the bacterial host cell described herein selected from the group of E. coli, Corynebacterium and Pseudomonas fluorescens. In some embodiments, the bacterial host cell described herein is an E. Coli cell, such as B. strains, in particular BL21 or BL21 (DE3). In some embodiments, the bacterial host cell described herein is a BL21(DE3) cell.

In certain embodiments, the invention relates to a disinfection solution comprising a polypeptide, the polypeptide comprising: a) an NIpC/P60 domain; and b) no CHAP domain of LysK.

The term "disinfection solution", as used herein, refers to a solution that can be used to destroy pathogens on nonliving surfaces. The nonliving surfaces can for example be the surface of a floor, a workspace, a tool or a device.

The inventors found that the polypeptide described herein can be used in a disinfection solution to destroy pathogens that are not destroyed by other disinfectants with comparable properties such as biological degradability.

Accordingly, the polypeptide described herein can be the primary active ingredient or may be combined with other active ingredients such as alcohol, chlorine, chlorine compounds, formaldehyde, glutaraldehyde, hydrogen peroxide, iodophors, orthophthalaldehyde, peracetic acid, peracetic acid, hydrogen peroxide, phenolics and/or quaternary ammonium compounds.

In certain embodiments, the invention relates to a packaging material comprising a polypeptide, the polypeptide comprising: a) an NIpC/P60 domain; and b) no CHAP domain of LysK.

In certain embodiments, the invention relates to a packaging material comprising LytF.

In some embodiments, the packaging material described herein is a packaging material comprising at least one selected from the group of plastic, paper and paperboard. In some embodiments, the packaging material described herein is a packaging material for food, medical devices and/or medication.

In some embodiments, the polypeptide described herein is incorporated in the packaging material. In some embodiments, the polypeptide described herein is layered on the packaging material.

Accordingly, the invention is at least in part based on the finding that packaging material comprising the polypeptide described herein can avoid contamination and prolong the stability of the packaged content.

In certain embodiments, the invention relates to a method for the production of a polypeptide, the method comprising the steps of: i) culturing the host cell of the invention; and ii) isolating the polypeptide comprising: a) an NIpC/P60 domain or a variant thereof having at least 80% sequence identity with the NlpC/P60 domain; and b) no CHAP domain of LysK.

In certain embodiments, the invention relates to a method for the production of a polypeptide, the method comprising the steps of: i) culturing the host cell of the invention; and ii) isolating LytF.

General methods for the production of polypeptides are known to the person skilled in the art (see e.g., Biotech, A. P., 2001, The recombinant protein handbook. Protein amplification and simple purification). The inventors found that the host cell of the invention can be used in a method for the particular efficient production of the polypeptide described herein.

In certain embodiments, the invention relates to a method for conservation of a product, the method comprising adding at least one polypeptide to a product, the polypeptide comprising: a) an NlpC/P60 domain; and b) no CHAP domain of LysK.

In some embodiments, the product described herein is a product selected from the group of food, medical device and/or medication. In some embodiments, the polypeptide described herein is added by mixing the polypeptide with a precursor of the product. In some embodiments, the polypeptide is added by layering the product with the polypeptide.

The inventors found that the polypeptide described herein can delay the expiration of products and/or avoid contamination thereof. Furthermore, the polypeptide described herein shows high stability, environmental tolerability, safety and/or selectivity for pathogens, if used in a method for conservation.

In certain embodiments, the invention relates to a method for prevention and/or containment of a contamination of a biotechnological process, the method comprising the steps of: i) combining with a nutrient a polypeptide, the polypeptide comprising: a) an NlpC/P60 domain; and b) no CHAP domain of LysK for use of preventing and/or containing a contamination; and ii) adding at least one cell to the nutrient medium to initiate and/or proceed the biotechnological process, wherein the cell is at least partially resistant to the polypeptide.

The term "biotechnological process", as used herein, refers to use of living organisms to develop and/or make products (see e.g. Harisha, S., 2008, Biotechnology procedures and experiments handbook. Laxmi Publications, Ltd.). In some embodiments, the living organisms are bioengineered living organisms.

The term "nutrient medium", as used herein, refers to a medium designed to support the growth of a population of microorganisms or cells via the process of cell proliferation. The nutrient medium can be a solid, a liquid, a semi-solid or a combination thereof. In some embodiments, the nutrient medium described herein comprises a carbon source such as glucose, water, salts and a source of amino acids and/or nitrogen. In some embodiments, the nutrient medium described herein is at least one nutrient medium selected from the group consisting of wort, agar and liquid nutrient medium.

The term "resistant to the polypeptide", as used herein, refers to the ability to substantially grow in the presence of the polypeptide, preferably to the ability to grow in presence of the polypeptide to a similar extent as in absence of the polypeptide.

In certain embodiments, the invention relates to the method of the invention, wherein the biotechnological process is the biotechnological production of at least one small molecule, nucleotide, or polypeptide.

"a," "an," and "the" are used herein to refer to one or to more than one (i.e., to at least one, or to one or more) of the grammatical object of the article.

"or" should be understood to mean either one, both, or any combination thereof of the alternatives.

"and/or" should be understood to mean either one, or both of the alternatives.

Throughout this specification, unless the context requires otherwise, the words "comprise", "comprises" and "comprising" will be understood to imply the inclusion of a stated step or element or group of steps or elements but not the exclusion of any other step or element or group of steps or elements.

The terms "about" or "approximately", as used herein, refer to "within 20%", more preferably "within 10%", and even more preferably "within 5%", of a given value or range.

The terms "include" and "comprise" are used synonymously. "preferably" means one option out of a series of options not excluding other options. "e.g." means one example without restriction to the mentioned example. By "consisting of' is meant including, and limited to, whatever follows the phrase "consisting of".

Reference throughout this specification to "one embodiment", "an embodiment", "a particular embodiment", "a related embodiment", "a certain embodiment", "an additional embodiment", "some embodiments", "a specific embodiment" or "a further embodiment" or combinations thereof means that a particular feature, structure or characteristic described in connection with the embodiment is included in at least one embodiment of the present invention. Thus, the appearances of the foregoing phrases in various places throughout this specification are not necessarily all referring to the same embodiment. Furthermore, the particular features, structures, or characteristics may be combined in any suitable manner in one or more embodiments. It is also understood that the positive recitation of a feature in one embodiment, serves as a basis for excluding the feature in a particular embodiment.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

The general methods and techniques described herein may be performed according to conventional methods well known in the art and as described in various general and more specific references that are cited and discussed throughout the present specification unless otherwise indicated. See, e.g., Sambrook et al., Molecular Cloning: A Laboratory Manual, 2d ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual, Cold Spring Harbor Laboratory Press, Cold Spring Harbor, N.Y. (1990).

While embodiments of the invention are illustrated and described in detail in the figures and foregoing description, such illustration and description are to be considered illustrative or exemplary and not restrictive. It will be understood that changes and modifications may be made by those of ordinary skill within the scope and spirit of the following claims. In particular, the present invention covers further embodiments with any combination of features from different embodiments described above and below.

### Brief description of Figures

Fig. 1: Growth curves for Bacillus subtilis cells (strain 3610) at 37°C.
Fig. 2: Growth curve of B. subtilis cells and addition of LytF.
Fig. 3: Structure of LytF: 5 LysM domains for binding the bacterial cell wall, and a C-terminal D, L-endopeptidase domain of the NIpC/P60 domain type (illustration adapted from SMART BLAST, EMBL).
Fig. 4: Schematic illustration of a NlpC/P60 domain.

### Examples

Aspects of the present invention are additionally described by way of the following illustrative non-limiting examples that provide a better understanding of embodiments of the present invention and of its many advantages. The following examples are included to demonstrate preferred embodiments of the invention. It should be appreciated by those of skill in the art that the techniques disclosed in the examples which follow represent techniques used in the present invention to function well in the practice of the invention, and thus can be considered to constitute preferred modes for its practice. However, those of skill in the art should appreciate, in light of the present disclosure that many changes can be made in the specific embodiments which are disclosed and still obtain a like or similar result without departing from the spirit and scope of the invention.

### Example 1

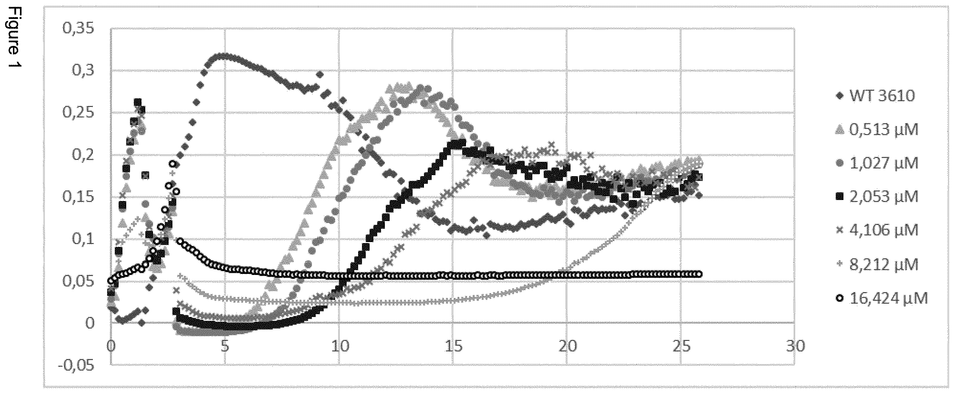

Growth curves for Bacillus subtilis cells (strain 3610) at 37°C. After 2.5 hours of growth (optical density from y-axis: 0.15), 1/100 volume of buffer (see legend: WT 3610) or purified LytF (see Fig. 1 legend for concentrations) was added. At the lowest concentration, renewed growth occurs after 5 hours following lysis; at the highest concentration, the lowest lysis occurs but inhibition of growth persists for over 20 hours (Fig. 1).

### Example 2

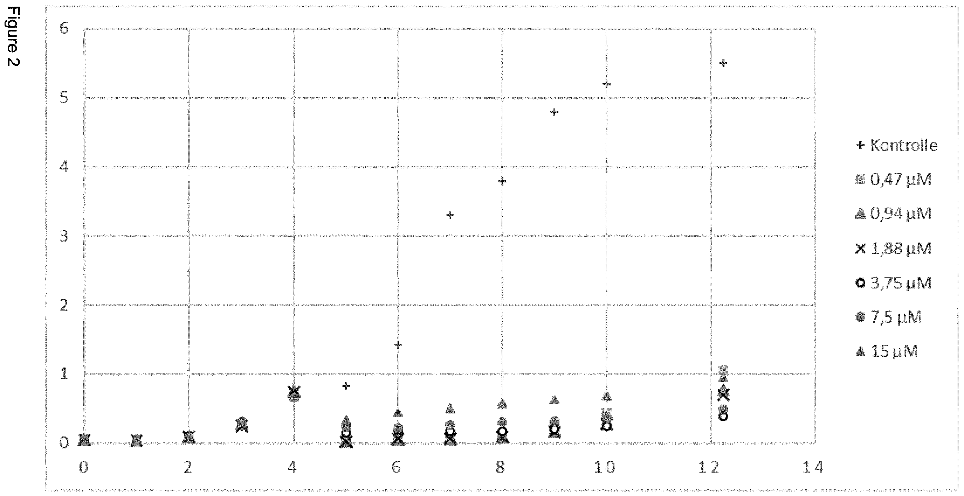

Growth curve of B. subtilis cells and addition of LytF after 4.5 h, at an optical density (y-axis) of 0.75. Growth occurs at concentrations up to 7.5 µM from c.a. 10 h, higher concentration of 15 µM has a smaller effect, so there might be an optimal effect concentration (Fig. 2).

### Example 3

The inventors found that a sample that was forgotten in the fridge for 6 months still showed substantial enzyme activity.

### Example 4

### Purification of LytF-His

A BL21 (DE3) culture, containing a lytF-His-pET24d+ vector SEQ ID NO: 10, was grown to an OD (600 nm) of 0.6. Then the culture was induced with lactose (1%). Overnight (around 16 h) the culture was incubated with lactose. The cells were harvested by centrifugation (5000xg, 20 min, 4°C). The cell pellet was resuspended in buffer A. Lysis of the cells was done with a microfluidizer. Lysed cells were centrifuged (40000xg, 20 min, 4°C) to get rid of insoluble cell parts. The supernatant with filtered an applied on a Ni-NTA-Column. Washing the column was done with at least 10 CV (column volumes) buffer A. The protein was eluted with at least 5 CV buffer B.

Afterwards the protein was purified by a SEC column.

| | Puffer A | Puffer B |
|---|---|---|
| HEPES | 20 mM | 20 mM |
| NaCl | 250 mM | 250 mM |
| MgCl₂ | 20 mM | 20 mM |
| KCI | 20 mM | 20 mM |
| Imidazol | 40 mM | 500 mM |

## Claims

1. A composition comprising a polypeptide for use as a medicament, the polypeptide comprising:
a) an NIpC/P60 domain; and
b) no CHAP domain of LysK.

2. The composition of claim 1 for use as an anti-infective medicament.

3. The composition for use of claim 2, wherein the anti-infective medicament is an antibiotic medicament.

4. The composition for use of claim 3, wherein the antibiotic medicament is used in the treatment and/or prevention of a gram-positive bacterial infection.

5. The composition of claim 1 for use in treatment of a disease or disorder of the skin, preferably wherein the disease or disorder of the skin is caused by at least one cause or disease selected from the group consisting of: acneiform eruptions, autoinflammatory syndromes, chronic blistering, conditions of the mucous membranes, conditions of the skin appendages, conditions of the subcutaneous fat, congenital anomalies, connective tissue diseases, abnormalities of dermal fibrous, abnormalities of elastic tissue, dermal and subcutaneous growths, dermatitis, drug eruptions, endocrine-related disease, epidermal nevi, epidermal neoplasms, epidermal cysts, erythemas, genodermatoses, lichenoid eruptions, lymphoid-related disease, melanocytic nevi, neoplasms, monocyte- and macrophage-related disease, mucinoses, neurocutaneous, noninfectious immunodeficiency-related disease, papulosquamous hyperkeratotic, pregnancy, pruritic, psoriasis, reactive neutrophilic disease, recalcitrant palmoplantar eruptions, metabolic disease, physical factors, urticaria, angioedema and vascular-related diseases.

6. The composition for use of claim 1 to 5, wherein the polypeptide is LytF or a derivative, variant or analogue thereof.

7. The composition for use of claims 1 to 6, wherein the composition is formulated for epicutaneous administration, preferably wherein the composition is formulated for a galenic form selected from the group consisting of: plaster, compress, topical solution, topical emulsion, topical suspension, cream, ointment, paste and gel.

8. A medical device comprising a polypeptide comprising:
a) an NIpC/P60 domain; and
b) no CHAP domain of LysK
preferably, wherein the medical device is a device selected from the group consisting of: implant, stent, catheter, intrauterine device, plaster, compress and dressing.

9. A polynucleotide comprising or consisting of a sequence as defined by the SEQ ID NO: 3 or a sequence having 25% sequence identity thereof, encoding at least one polypeptide comprising:
a) an NlpC/P60 domain; and
b) no CHAP domain of LysK,
preferably LytF.

10. A vector and/or host cell comprising the polynucleotide of claim 9.

11. The host cell of claim 10, wherein the host cell is a bacterial cell.

12. A disinfection solution and/or a packaging material comprising a polypeptide, the polypeptide comprising:
a) an NIpC/P60 domain; and
b) no CHAP domain of LysK.

13. A method for the production of a polypeptide, the method comprising the steps of:
i) culturing the host cell of claim 10 or 11; and
ii) isolating the polypeptide comprising:
a) an NIpC/P60 domain; and
b) no CHAP domain of LysK.

14. A method for conservation of a product, the method comprising adding at least one polypeptide to a product, the polypeptide comprising:
a) an NlpC/P60 domain; and
b) no CHAP domain of LysK.

15. A method for prevention and/or containment of a contamination of a biotechnological process, the method comprising the steps of:
i) combining with a nutrient a polypeptide, the polypeptide comprising:
a) an NIpC/P60 domain; and
b) no CHAP domain of LysK
for use of preventing and/or containing a contamination; and
ii) adding at least one cell to the nutrient medium to initiate and/or proceed the biotechnological process, wherein the cell is at least partially resistant to the polypeptide,
preferably wherein the biotechnological process is the biotechnological production of at least one small molecule, nucleotide, or polypeptide.
